# EUROPEAN PATENT APPLICATION

(11) **EP 3 741 422 A1**
(43) Date of publication of application: **25.11.2020**
(21) Application number: 18900547.3
(22) Date of filing: 26.01.2018
(51) Int. Cl.: A61M 39/22, A61M 39/24

(54) **THREE-WAY DEVICE FOR ANTI-BACKFLOW INFUSION AND DOSING**

(30) Priority: 15.01.2018 CN 201820061963 U; 15.01.2018 CN 201810035475
(71) Applicant: Shanghai K&G International Co., Ltd., Shanghai 200120 (CN)
(72) Inventor: ZHANG, Jing, Shanghai 200120 (CN)
(74) Representative: Dr. Gassner & Partner mbB
(86) International application number: PCT/CN2018/074366
(87) International publication number: WO 2019/136779

(57) **Abstract**

A three-way device for anti-backflow infusion and dosing, comprising a three-way valve body (1), the two ends of the three-way valve body (1) acting as liquid inlets, and an interface of the middle of the three-way valve body (1) acting as a liquid outlet; and a non-return mechanism is installed inside the three-way valve body (1). The non-return mechanism comprises a reverse-stop elastic tube (3) and a connecting piece (4), the connecting piece (4) being mounted with a sliding fit in the middle in the three-way valve body (1), the two ends of the connecting piece (4) respectively being connected to one end of the reverse-stop elastic tube (3), and the other end of each reverse-stop elastic tube (3) being closed, and abutting the inner end of a connecting base (2). A gap is kept between the reverse-stop elastic tube (3) and the inner wall of the three-way valve body (1). Each liquid inlet of the three-way device can be connected to a syringe and, when a liquid drug is injected by any one syringe, an infusion path is automatically opened by means of compressing the closed end of the related reverse-stop elastic tube (3), without the need for any closing and opening operation, thereby preventing misoperation. Leakage, overflow, and backflow will not occur when in use, ensuring the safety of the infusion and the injection and aspiration performed during the infusion process.

## Description

### TECHNICAL FIELD

The present disclosure relates to a three-way device for anti-backflow infusion and dosing, which pertains to the field of medical apparatus and instruments.

### BACKGROUND

The existing three-way devices for infusion have a poorer one-way non-return effect and require switches to perform the non-return operation. When in use, the three-way devices for infusion are connected to hoses, making it difficult to operate the switches, leading to leakage, overflow and backflow, and thus causing potential safety hazards in the processes of infusion or dosing.

### SUMMARY

To overcome defects of the existing technologies, the present disclosure provides a three-way device for anti-backflow infusion and dosing.

Technical solutions of the present disclosure are as below. A three-way device for anti-backflow infusion and dosing includes a three-way valve body, two ends of the three-way valve body act as liquid inlets, and on each of the liquid inlets there is provided with an arcuate connecting base in a fit connection with an outlet of a syringe. An interface in a middle of the three-way valve body acts as a liquid outlet, and is provided with a hose connecting base. A non-return mechanism is mounted inside the three-way valve body.

The non-return mechanism includes a reverse-stop elastic tube and a connecting piece, the connecting piece is mounted with a sliding fit in the middle in the three-way valve body, two ends of the connecting piece respectively are connected to one end of the reverse-stop elastic tube, and the other end of each reverse-stop elastic tube is closed and abuts an inner end the connecting base as a valve core. A gap is kept between the reverse-stop elastic tube and an inner wall of the three-way valve body.

A middle part of the connecting piece is a disc body, and an outer periphery surface of the disc body is in a sliding fit with the inner wall of the three-way valve body. Two ends of the disc body are respectively provided with a reduced-diameter stub, and an open end of the reverse-stop elastic tube is sleeved on the reduced-diameter stub.

A tube wall of the reverse-stop elastic tube is arched in a circular-arc shape or in a triangular shape toward an outer periphery. A closed end of the reverse-stop elastic tube is in a cylinder shape, whose diameter is greater than diameters of two ends of the tube wall.

The connecting base is L-shaped.

The connecting base is a short straight-line connecting base and is homoaxial with respect to the three-way valve body.

The connecting base is a long straight-line connecting base and is homoaxial with respect to the three-way valve body.

The three-way valve body is made from a transparent material, and the reverse-stop elastic tube is made of a medical grade rubber.

Advantages of the present disclosure are as below. The three-way device for anti-backflow infusion and dosing may be used singly, or a plurality of three-way devices for anti-backflow infusion and dosing may be used in parallel connection. Each liquid inlet of the three-way device may be connected to a syringe and, when a liquid drug is injected by any one syringe, an infusion path is automatically opened by means of compressing the closed end of the related reverse-stop elastic tube, without the need for any closing and opening operation, thereby preventing misoperation. Leakage, overflow, and backflow are prevented when in use, ensuring the safety of the infusion and the injection and aspiration performed during the infusion process.

### BRIEF DESCRIPTION OF THE DRAWINGS

FIG. 1 is a front view of an overall structure according to a first embodiment of the present disclosure;
FIG. 2 is a left (right) view of FIG. 1;
FIG. 3 is an A-A sectional view of FIG. 2;
FIG. 4 is an exploded view according to the previous embodiment;
FIG. 5 is a sectional structural front view according to a second embodiment of the present disclosure;
FIG. 6 is a sectional structural front view according to a third embodiment of the present disclosure; and
FIG. 7 is a schematic diagram according to an application example of the present disclosure.

### DETAILED DESCRIPTION

The present disclosure is further described in detail below with reference to specific embodiments, and advantages and characteristics of the present disclosure will become more apparent with the description. However, these embodiments are merely exemplary rather than limiting the scope of the present disclosure. Those skilled in the art should realize that various modifications or substitutions of technical solutions of the present disclosure may be made in form and in detail without departing from the spirit and scope of the present disclosure, and these modifications and substitutions fall within the protection scope of the present disclosure.

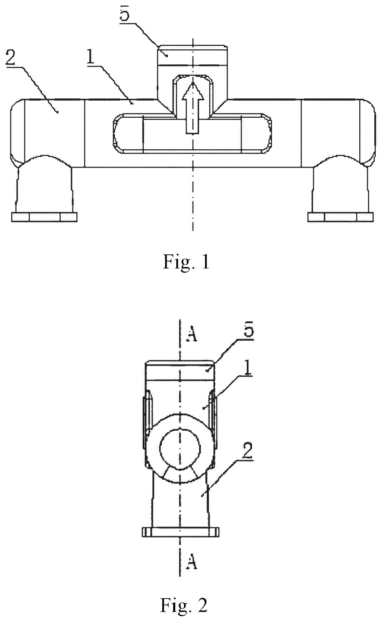

Referring to FIG. 1-FIG. 6, a basic structure of a three-way device for anti-backflow infusion and dosing provided by the present disclosure includes a three-way valve body 1, two ends of the three-way valve body 1 act as liquid inlets, and on each of the liquid inlets there is provided with a connecting base 2 in a fit connection with an outlet of a syringe. An interface in the middle of the three-way valve body 1 acts as a liquid outlet, and is provided with a hose connecting base 5. A non-return mechanism is mounted inside the three-way valve body 1.

The three-way valve body 1 is connected to the connecting base 2 and the hose connecting base 5 by a threaded connection (a gasket may be additionally provided), adhesive bonding, heat sealing connection or interference connection.

The non-return mechanism includes two reverse-stop elastic tubes 3 and one connecting piece 4, the connecting piece 4 is mounted with a sliding fit in the middle in the three-way valve body 1, two ends of the connecting piece 4 respectively are sleeved on one end of one reverse-stop elastic tube 3, and the other end of the reverse-stop elastic tube 3 is closed, and the closed end of the reverse-stop elastic tube 3 serves as a valve core (an on-off member of a valve) and abuts the inner end (equivalent to a valve seat) of the connecting base 2. A gap is kept between the reverse-stop elastic tube 3 and the inner wall of the three-way valve body 1. In a stationary state, the closed ends of the two reverse-stop elastic tubes 3 abut the inner end the connecting base 2 to close the two liquid inlets of the three-way device.

The three-way valve body 1 is made from a transparent material such as a transparent plastic, and the reverse-stop elastic tube is made of a medical grade rubber.

A middle part of the connecting piece 4 is a disc body 41, and an outer periphery surface of the disc body 41 is in a sliding fit with the inner wall of the three-way valve body 1. Two ends of the disc body 41 are respectively provided with a reduced-diameter stub 42, and an open end of the reverse-stop elastic tube 3 is sleeved on the reduced-diameter stub 42.

A tube wall 32 of the reverse-stop elastic tube 3 is arched in a circular-arc shape (or in a triangular shape) toward an outer periphery. A closed end 31 of the reverse-stop elastic tube 3 is in a cylinder shape, whose diameter is greater than diameters of two ends of the tube wall 32 (essentially equal to an outer diameter of the arched tube wall 32). The arched tube wall 32 facilitates its extension in an axial direction.

According to the first embodiment of the present disclosure as shown in FIG. 1-FIG. 4, the connecting bases 2 at the two ends of the three-way valve body 1 are L-shaped, and the interface may fit with the outlet of the syringe.

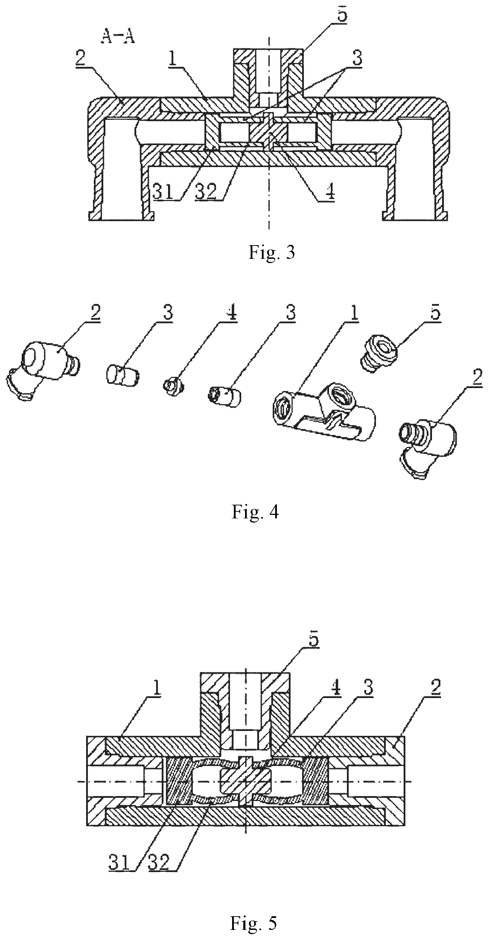

The connecting base 2 of the present disclosure may also employ a short straight-line connecting base of the second embodiment as shown in FIG. 5, and is homoaxial with respect to the three-way valve body 1. The interface of the short straight-line connecting base is mainly used for docking with a connecting tube, and may not fit with the outlet of the syringe.

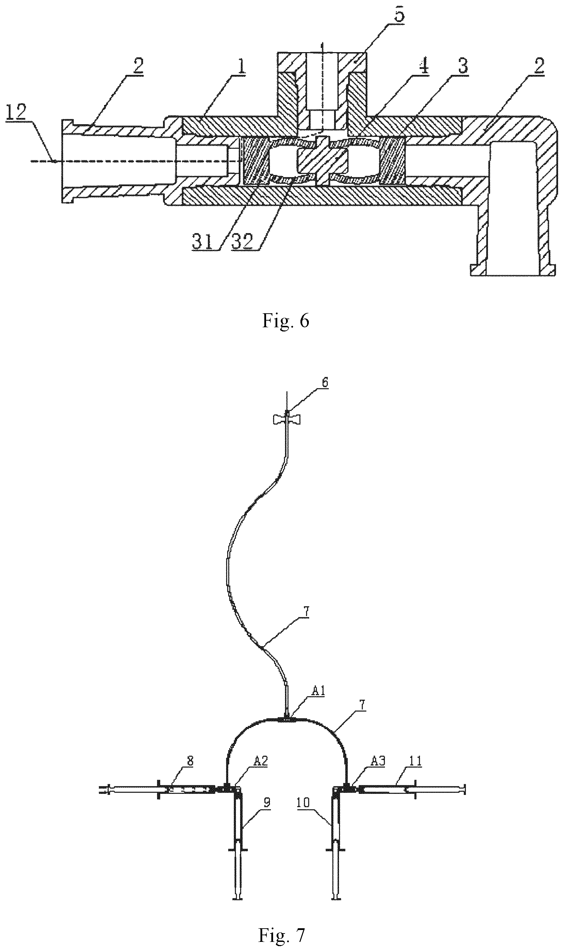

The connecting base 2 of the present disclosure may also employ a long straight-line connecting base (the interface may fit with the outlet of the syringe) of the third embodiment as shown in FIG. 6, and is homoaxial with respect to the three-way valve body 1. The other connecting base 2 in this embodiment is an L-shaped connecting base as that in the first embodiment.

The connecting bases 2 having three structures in the above three embodiments may be combined arbitrarily.

FIG. 7 shows an application example of three three-way devices A1, A2 and A3 of the present disclosure. The liquid outlet of the first three-way device A1 is connected to an infusion needle 6 through a hose 7, and two liquid inlets (connecting bases 2) of the three-way device A1 are respectively connected to the liquid outlets (the hose connecting bases 5) of the other two three-way devices A2 and A3 through the hose 7. Four liquid inlets of two three-way devices A2 and A3 are docked with injection ports of four syringes 8-11 at the same time. In actual use, the four syringes may inject drugs simultaneously or separately. When any one of the syringes (referring to FIG. 6 and FIG. 7) injects drugs, pressure outputted by the syringe is applied to the closed end of the reverse-stop elastic tube 3 in the liquid inlet at the left end. That is, the closed end of the reverse-stop elastic tube 3 is compressed inward (rightwards), such that a gap is kept between the closed end of the reverse-stop elastic tube 3 and the connecting bases 2 at the left end. In this way, an infusion channel (as shown by the dotted line 12 with arrows in FIG. 6) is formed in sequence from the liquid inlet at the left end, the gap between the inner end of the connecting base 2 at the left end and the closed end of the reverse-stop elastic tube 3, the gap between the reverse-stop elastic tube 3 and the inner wall of the three-way valve body 1 to the liquid outlet, thereby completing the injection of a liquid drug. Dosing principles of the other three syringes are the same. The liquid inlet where no drug is injected is in a closed state due to absence of inward pressure, and thus diversion of the liquid drug will not occur. No operation of the three-way device needs to be conducted in the dosing processes of a plurality of liquid inlets, and thus it is not prone to operation errors, liquid leakage and liquid backflow. However, an ordinary three-way device needs to turn its switch when in use. In many combinations, it is prone to problems of difficult operation or misoperation.

When the liquid drug is dosed to both sides of the three-way device for anti-backflow infusion and dosing at the same time, the connecting piece 4 basically does not produce displacement, the closed ends of the two reverse-stop elastic tubes 3 are compressed by force, and liquid drug channels are formed at the liquid inlets at two ends and at the liquid outlet of the three-way valve body 1.

When the reverse-stop elastic tube 3 is under a state where no pressure is applied, the liquid drug channel is in a closed state. If the liquid drug enters from the liquid outlet in the reverse direction, the tube wall of the reverse-stop elastic tube 3 is compressed in the radial direction, forcing the reverse-stop elastic tube 3 to expand in the axial direction to push the closed end of the reverse-stop elastic tube 3 and the inner end of the connecting base 2 to be closed more tightly. The greater the pressure is, the better the leakproofness is, such that the liquid is prevented from flowing out in the reverse direction.

The present invention may also aspirate liquid drug at the liquid outlet, such that the liquid drug enters through the liquid inlets at two ends. The liquid drug applies pressure to the reverse-stop elastic tubes 3 at two ends to form the liquid drug channel to complete the aspiration.

The present invention may be applicable to liquid infusion or dosing in various medical occasions, and especially may be used in combination with ordinary infusion devices widely used at present, which not only can complete ordinary infusion, but also can ensure the liquid to flow unidirectionally to prevent venous blood from flowing back to an infusion set. It especially has significant effects on the infusion of acute and severe patients, and also can be connected with a plurality of dosing injection systems filled with different liquid drugs for treatment.

## Claims

1. A three-way device for anti-backflow infusion and dosing, comprising a three-way valve body, two ends of the three-way valve body acting as liquid inlets, and a connecting base being provided on each of the liquid inlets; an interface in a middle of the three-way valve body acting as a liquid outlet, and being provided with a hose connecting base; and a non-return mechanism being mounted inside the three-way valve body.

2. The three-way device for anti-backflow infusion and dosing according to claim 1, wherein the non-return mechanism comprises a reverse-stop elastic tube and a connecting piece, the connecting piece is mounted with a sliding fit in the middle in the three-way valve body, two ends of the connecting piece respectively are connected to one end of the reverse-stop elastic tube, and the other end of each reverse-stop elastic tube is closed and abuts an inner end the connecting base as a valve core, and a gap is kept between the reverse-stop elastic tube and an inner wall of the three-way valve body.

3. The three-way device for anti-backflow infusion and dosing according to claim 2, wherein a middle part of the connecting piece is a disc body, and an outer periphery surface of the disc body is in a sliding fit with the inner wall of the three-way valve body; and two ends of the disc body are respectively provided with a reduced-diameter stub, and an open end of the reverse-stop elastic tube is sleeved on the reduced-diameter stub.

4. The three-way device for anti-backflow infusion and dosing according to claim 2, wherein a tube wall of the reverse-stop elastic tube is arched in a circular-arc shape or in a triangular shape toward an outer periphery; and a closed end of the reverse-stop elastic tube is in a cylinder shape, whose diameter is greater than diameters of two ends of the tube wall.

5. The three-way device for anti-backflow infusion and dosing according to claim 1, wherein the connecting base is L-shaped.

6. The three-way device for anti-backflow infusion and dosing according to claim 1, wherein the connecting base is a short straight-line connecting base and is homoaxial with respect to the three-way valve body.

7. The three-way device for anti-backflow infusion and dosing according to claim 1, wherein the connecting base is a long straight-line connecting base and is homoaxial with respect to the three-way valve body.

8. The three-way device for anti-backflow infusion and dosing according to claim 1, wherein the three-way valve body is made from a transparent material, and the reverse-stop elastic tube is made of a medical grade rubber.
